# EUROPEAN PATENT SPECIFICATION

(11) **EP 3 981 373 B1**
(45) Date of publication and mention of the grant of the patent: **24.01.2024**
(21) Application number: 20200574.0
(22) Date of filing: 07.10.2020
(51) Int. Cl.: A61G 13/02, A61B 5/00, A61H 31/00

(54) **SURGICAL TABLE AND METHOD FOR DETECTING CARDIOPULMONARY RESUSCITATION BY THE SURGICAL TABLE**
OPERATIONSTISCH UND VERFAHREN ZUR ERFASSUNG DER KARDIOPULMONALEN WIEDERBELEBUNG DURCH DEN OPERATIONSTISCH
TABLE CHIRURGICALE ET PROCÉDÉ DE DÉTECTION DE RÉANIMATION CARDIO-PULMONAIRE PAR LA TABLE CHIRURGICALE

(43) Date of publication of application: 13.04.2022
(73) Proprietor: Baxter Medical Systems GmbH + Co. KG, 07318 Saalfeld (DE)
(72) Inventor: JÄGER, Matthias, Batesville, IN 47006 (US)
(74) Representative: Loustalan, Paul William

(56) References cited:
- CN-A- 103 284 858
- CN-A- 110 368 286
- US-A1- 2020 155 402

## Description

The invention relates to a surgical table and a method for detecting cardiopulmonary resuscitation by the surgical table, in particular, to a surgical table and a method for detecting cardiopulmonary resuscitation by the surgical table in an automatic manner.

Nowadays, there is a corporate strategy of connected care. This means that relevant data of a patient are collected, e.g., during hospitalization and provided to medical staff if necessary.

However, collecting of some of these patient data is cumbersome and, therefore, it is not logged immediately so that there is the risk that it is omitted.

CN1 10368286A provides a wireless cardiopulmonary resuscitation (CPR) guidance device, which belongs to the field of medical apparatus. The main hardware parts of said wireless CPR guidance device include a logic control module, a chest compression detection module which uses accelerometers, an information display module, a voice prompt module, a wireless data communication module, and a power management module. Said CPR guidance device can achieve automatic power management according to the actual chest compression movement; and guide the completion of the first aid process according to the standard CPR process; the first aid process can feedback on the actual resuscitation behaviour, making CPR more standard and improving the quality of CPR.

US2020/155402A1 provides a patient weight measuring system which includes a patient table, load sensors, and a control circuit.

Therefore, the object underlying the invention is to provide an apparatus and a method for automatically collecting patient's data, in particular, occurrence of a cardiopulmonary resuscitation during a surgical invention.

The object is achieved by a surgical table according to claim 1 and a method according to claim 10. Advantageous further developments are included in the dependent claims.

According to an aspect of the invention, a surgical table comprises a base, a tabletop attached to the base, a force sensor, and a controller. The force sensor is configured to detect a force exerted to the tabletop and to transmit the force to the controller, and the controller is configured to evaluate the force exerted to the tabletop and to execute a predefined routine for determining cardiopulmonary resuscitation for a patient lying on the tabletop by distinguishing forces caused by the cardiopulmonary resuscitation from other forces exerted to the tabletop.

By the surgical table having these features, cardiopulmonary resuscitation can be gathered as patient's data in an automatic manner without any further manual intervention and in a reliable manner since other forces not caused by the cardiopulmonary resuscitation are not considered by the routine for determining cardiopulmonary resuscitation.

In a further advantageous implementation of the surgical table, the predefined routine is configured to stop evaluation during motion of the surgical table.

Due to this feature, detection of a pattern of forces caused by the motion of the surgical table which might be similar to a pattern of forces caused by cardiopulmonary resuscitation can be suppressed so that it is not considered as being the cardiopulmonary resuscitation.

In a further advantageous implementation, the predefined routine is configured to determine exerted forces as forces of the cardiopulmonary resuscitation when force peaks values are within a predefined force peak value range and a force peak interval is within a predefined force peak interval range.

Since the force peaks values caused by cardiopulmonary resuscitation are usually similar and a frequency of the force peaks is almost constant, due to the definition of the force peak value range and the force peak interval range, cardiopulmonary resuscitation can be determined reliably.

In a further advantageous implementation, the predetermined force peak value range is defined by an upper limit value being 5% more than a first detected force peak value and a lower limit value being 5% less of the first detected force peak value.

The definition of the force peak value range in such a manner enhances the reliability of the determination of the cardiopulmonary resuscitation.

In a further advantageous implementation, the predetermined force peak interval is formed by a range of 80 to 120 force peaks/minute.

The definition of the force peak interval range in such a manner enhances the reliability of the determination of the cardiopulmonary resuscitation.

In a further advantageous implementation, the force sensor is arranged in the tabletop.

When the force sensor is arranged in the tabletop, a direct detection of the exerted force without any influence of the inertia of the tabletop is possible in order to enhance the detection of the force exerted by the cardiopulmonary resuscitation.

In another advantageous implementation, the force sensor is arranged in the base of the surgical table.

When being arranged in the base of the surgical table where usually the controller of the surgical table is located, connecting of the force sensor and the controller can easily be performed and it can be considered as highly reliable.

In a further advantageous implementation, the force sensor is configured as a sensor additionally providing load data of a quasi-static load of the tabletop to the controller.

When the force sensor being provided for additionally supplying quasi-static load data to the controller is used, no additional force sensor for the determination of the cardiopulmonary resuscitation is necessary in order to reduce effort and costs.

In a further advantageous implementation, the controller is configured to store occurrence of the cardiopulmonary resuscitation.

When the controller is configured to store the occurrence of the cardiopulmonary resuscitation, the medical staff is exempt from the need of manually logging the cardiopulmonary resuscitation.

According to a further aspect of the invention, a method for detecting cardiopulmonary resuscitation by a surgical table includes the steps: detecting several consecutive force peaks by the force sensor and determining the consecutive force peaks as cardiopulmonary resuscitation if the force peak values are within a predefined force peak value range and the force peaks are within a predefined force peak interval range.

By this method, cardiopulmonary resuscitation can be gathered as patient's data in an automatic manner without any further manual intervention.

In a further advantageous implementation of the method, the predetermined force peak value range is defined by an upper limit value being 5% more than a first detected force peak value and a lower limit value being 5% less than the first detected force peak value .

The execution of the method with the force peak value range in such manner enhances the reliability of the determination of the cardiopulmonary resuscitation.

In a further advantageous implementation of the method, an end of the cardiopulmonary resuscitation is determined if, within a predefined interval after a last force peak determined as being a force peak of the cardiopulmonary resuscitation, there are no force peaks, or the consecutive force peaks are outside the predefined force peak value range and/or outside the predefined force peak interval range.

By these features of the method, cardiopulmonary resuscitation, in particular, a duration of the cardiopulmonary resuscitation can be gathered as patient's data in an automatic manner without any further manual intervention since also the end of the cardiopulmonary resuscitation is detected.

In a further advantageous implementation of the method, occurrence of the cardiopulmonary resuscitation is stored by the controller.

When storing the occurrence of the cardiopulmonary resuscitation by the controller, the medical staff is exempt from the need of manually logging the cardiopulmonary resuscitation.

In a further advantageous implementation of the method, a duration of the cardiopulmonary resuscitation is detected and stored by the controller.

When also the duration of the cardiopulmonary resuscitation is detected and stored, severity of an incident of a cardiac arrest can be estimated retrospectively.

In a further advantageous implementation of the method, the occurrence of the cardiopulmonary resuscitation and/or a duration of the cardiopulmonary resuscitation are/is transferred to a hospital's Electronic Medical Record system.

When this data is transferred to the Electronic Medical Record system, the data is available for any necessary use by the hospital's staff.

Below, the invention is elucidated by means of embodiments referring to the attached drawings.

In particular,
- Fig. 1: shows a principal view of a first embodiment of a surgical table according to the invention;
- Fig. 2: shows a principal view of a second embodiment of the surgical table according to the invention;
- Fig. 3: shows a diagram including an example of a force curve of a force caused by cardiopulmonary resuscitation; and
- Fig. 4: shows a diagram including an example of a force curve of a force not caused by the cardiopulmonary resuscitation.

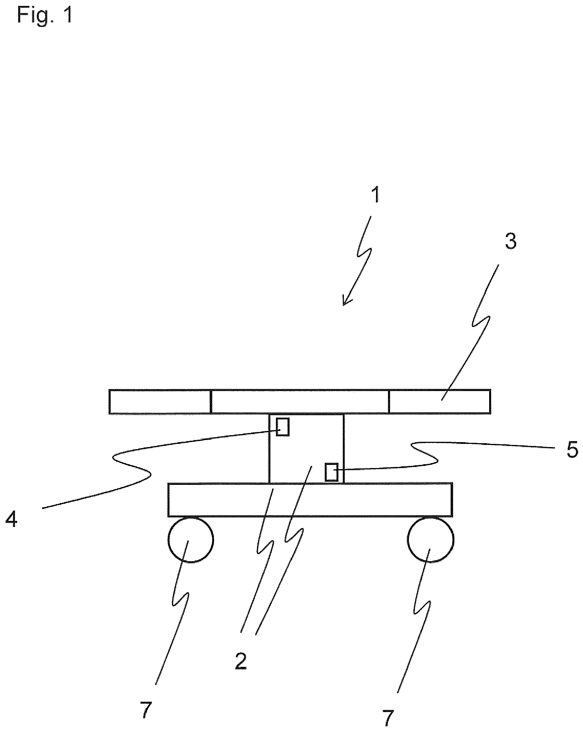

**Fig. 1** shows a principal view of a first embodiment of a surgical table 1 according to the invention. The surgical table 1 comprises a base 2 and a tabletop 3 attached to the base 2. The surgical table 1 is configured as a movable surgical table having castors 7 for being movable.

The surgical table 1 further comprises a force sensor 4 and a controller 5. The force sensor 4 is arranged in the base of the surgical table 1.

The force sensor 4 is further configured to detect a force exerted to the tabletop 3. This detected force can be a force caused by a patient lying on the tabletop 3, by external forces impacting onto the tabletop 3, or by inertia force when the surgical table 1 is, e.g., moved across a door sill. Moreover, the force sensor 4 is configured as a sensor additionally providing quasi-static load data of the tabletop 3 to the controller 5. This means that the force sensor 4 can be used for two different tasks, namely for the detection of the force caused by the cardiopulmonary resuscitation and for the detection of, e.g., the weight of the patient for detecting overload of the surgical table 1. In an alternative embodiment, the first sensor 4 is provided only for detecting the forces due to the cardiopulmonary resuscitation.

The controller 5 is configured to control drives (not shown) of the surgical table 1 to move the tabletop 3 with respect to the base 2 in order to perform a height adjustment of the tabletop 3 in order to tilt the tabletop 3. Further, the controller 5 is configured to evaluate the force exerted to the tabletop 3.

Furthermore, the controller 5 is configured to execute a predefined routine for determining cardiopulmonary resuscitation for a patient lying on the tabletop 3. This predefined routine is configured to distinguish forces caused by the cardiopulmonary resuscitation from other forces exerted to the tabletop 3.

Moreover, the controller 5 is configured to store occurrence and duration of the cardiopulmonary resuscitation. In alternative embodiments, the controller 5 does not store the occurrence and duration of the cardiopulmonary resuscitation but transfers the occurrence and duration to, e.g., a hospital's Electronic Medical Record system.

The predefined routine is configured to stop evaluation during motion of the surgical table 1. In an alternative embodiment, the routine does not stop evaluation during motion of the surgical table 1 but, e.g., it performs the evaluation but it does not store or transfer the result of the evaluation.

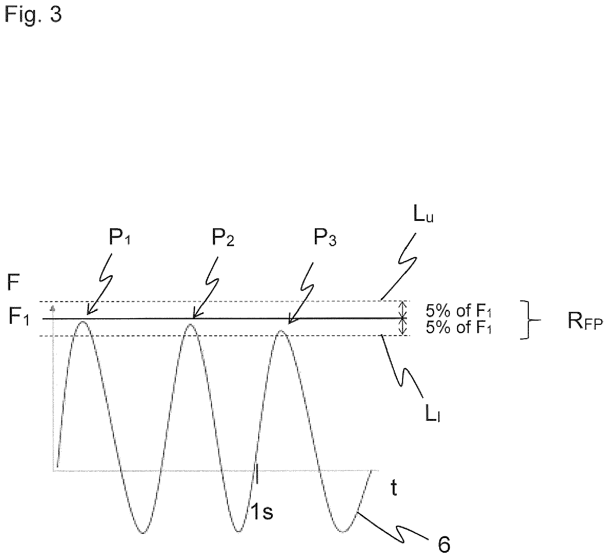

Furthermore, the predefined routine is configured to determine the exerted forces as forces of the cardiopulmonary resuscitation when force peaks values are within a predefined force peak value range R_{FP} (see Fig. 3) and a force peak interval is within a predefined force peak interval range.

In alternative embodiments, the routine is configured not to be focused on the force peaks but, e.g., on other portions of the force curve.

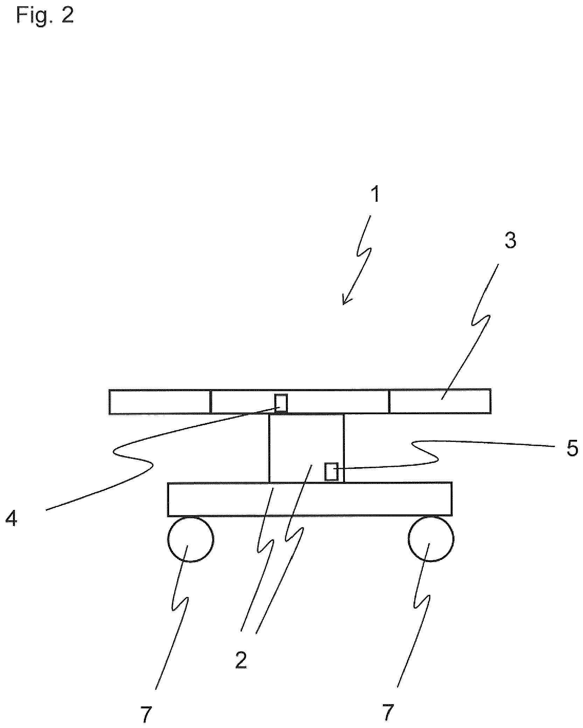

Fig. 2 shows a principal view of a second embodiment of the surgical table 1 according to the invention.

The surgical table 1 according to the second embodiment differs from the surgical table 1 according to the first embodiment in that the force sensor 4 is not arranged in the base 2 but it is arranged in the tabletop 3.

**Fig. 3** shows a diagram including an example of a force curve 6 of a force caused by the cardiopulmonary resuscitation. On the abscissa, an elapsed time t is plotted and, on the ordinate, a force value F is plotted.

The predetermined force peak value range R_{FP} is defined by an upper limit value Lᵤ being 5% more than a first detected force peak value F₁ and a lower limit value Lₗ being 5% less than the first detected force peak value F₁. In this embodiment, the predetermined force peak interval is formed by a range of 80 to 120 force peaks/minute. In alternative embodiments, the ranges are defined as having other limits.

As to be seen in Fig. 3, all values of the force peaks P₁, P₂, P₃ of the force curve 6 are within the range R_{FP}. Furthermore, as to be seen from the time course, two oscillations are detected within 1 second which corresponds to a force peak interval of 120 force peaks/minute. Thus, the exerted force is determined as force of the cardiopulmonary resuscitation.

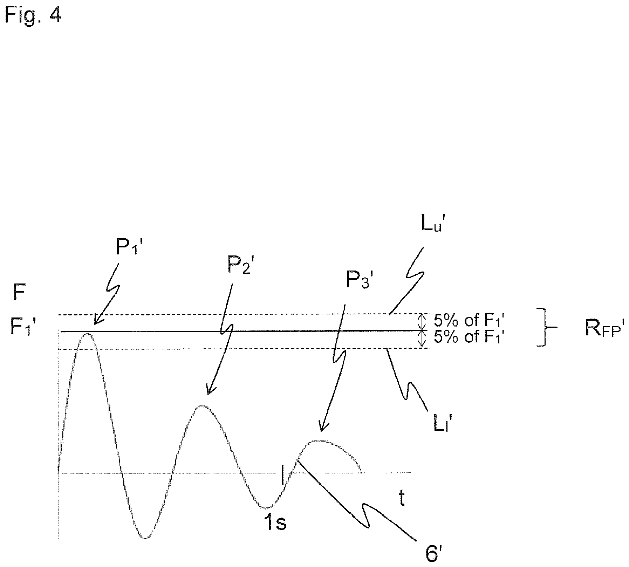

**Fig. 4** shows a diagram including an example of a force curve 6' of a force not caused by the cardiopulmonary resuscitation.

Also here, on the abscissa, the elapsed time t is plotted on and, on the ordinate, the force value F is plotted. Also, the indications concerning the upper limit value Lᵤ' being 5% more than the first detected force peak value F₁' and the lower limit value Lₗ' being 5% less than the first detected force peak value F₁' are plotted corresponding to Fig. 3.

As to be seen in Fig. 4, the value of the consecutive force peaks P₂', P₃' following the first force peak P₁' are not within the range R_{FP}' having the upper limit value Lᵤ' and the lower limit value Lₗ'. Therefore, the exerted force is not determined as being a force of a cardiopulmonary resuscitation.

In use, several consecutive force peaks P₁, P₂, P₃, P₁', P₂', P₃' are detected by the force sensor 4. The consecutive force peaks P₁, P₂, P₃, P₁', P₂', P₃' are determined as cardiopulmonary resuscitation if their force peak values are within the predefined force peak value range R_{FP}, R_{FP} '.

The predefined force peak value range R_{FP}, R_{FP} ' is defined by the upper limit value being 5% more than the first detected force peak F₁, F₁' and by the lower limit value being 5% less than the first detected force peak value F₁, F₁'. As already mentioned above, alternatively, the ranges are defined as having other limits or the routine may be configured not to be focused on the force peaks but on other portions of the force curve.

An end of the cardiopulmonary resuscitation is determined if, within a predefined interval after the last force peak determined as being a force peak of the cardiopulmonary resuscitation, there are no force peaks, or the force peak values of the consecutive force peaks are outside the predefined force peak value range R_{FP}, R_{FP} ' and/or outside the predefined force peak interval. Alternatively, an end of the cardiopulmonary resuscitation is not determined.

The occurrence of the cardiopulmonary resuscitation is stored by the controller 5. Alternatively, the occurrence of the cardiopulmonary resuscitation is not stored by the controller 5 but, e.g., transferred to another database.

Except the occurrence of the cardiopulmonary resuscitation, a duration of the cardiopulmonary resuscitation is detected and stored by the controller 5. Alternatively, the duration is not stored by the controller 5, but, e.g., transferred to another database.

Optionally, the occurrence of the cardiopulmonary resuscitation and/or the duration of the cardiopulmonary resuscitation are/is transferred to a hospital's Electronic Medical Record System.

While the invention has been illustrated and described in detail in the drawings and the foregoing description, such illustration and description are to be considered illustrative or exemplary and not restrictive. The invention is not limited to the disclosed embodiments. From reading the present disclosure, other modifications will be apparent to a person skilled in the art. Such modifications may involve other features, which are already known in the art and may be used instead of or in addition to features already described herein. In the claims, the word "comprising" does not exclude other elements or steps, and the indefinite article "a" or "an" does not exclude a plurality.

## Claims

1. A surgical table (1) comprising
a base (2),
a tabletop (3) attached to the base (2),
a force sensor (4), and
a controller (5), wherein
the force sensor (4) is configured to detect a force exerted to the tabletop (3) and to transmit the force to the controller (5), and
the controller (5) is configured to evaluate the force exerted to the tabletop (3) and to execute a predefined routine for determining cardiopulmonary resuscitation for a patient lying on the tabletop (3) by distinguishing forces caused by the cardiopulmonary resuscitation from other forces exerted to the tabletop (3).

2. The surgical table (1) of claim 1, wherein
the predefined routine is configured to stop evaluation during motion of the surgical table (1).

3. The surgical table (1) of claim 2, wherein
the predefined routine is configured to determine exerted forces as forces of the cardiopulmonary resuscitation when force peaks values are within a predefined force peak value range (R_{FP}) and a force peak interval is within a predefined force peak interval range.

4. The surgical table (1) of claim 3, wherein
the predetermined force peak value range (R_{FP}) is defined by an upper limit value (Lᵤ) being 5% more than a first detected force peak value and a lower limit value (L_{I}) being 5% less than the first detected force peak value (F₁).

5. The surgical table (1) of claim 3 or 4, wherein
the predetermined force peak interval range is formed by a range of 80 to 120 force peaks/minute.

6. The surgical table (1) of any preceding claim, wherein
the force sensor (4) is arranged in the tabletop (3).

7. The surgical table (1) of anyone of claims 1 to 5, wherein
the force sensor (4) is arranged in the base (2).

8. The surgical table (1) of any preceding claim, wherein
the force sensor (4) is configured as a sensor additionally providing load data of the tabletop (3) to the controller (5).

9. The surgical table (1) of any preceding claim, wherein
the controller (5) is configured to store occurrence of the cardiopulmonary resuscitation.

10. A method for detecting cardiopulmonary resuscitation by a surgical table (1) of any preceding claim, including the steps:
detecting several consecutive force peaks (P₁, P₂, P₃, P₁', P₂', P₃') by the force sensor (4); and
determining the consecutive force peaks (P₁, P₂, P₃) as cardiopulmonary resuscitation if the force peak values are within a predefined force peak value range (R_{FP}) and the force peaks are within a predefined force peak interval range.

11. The method of claim 10, wherein
the predefined force peak value range (R_{FP}) is defined by an upper limit value (Lᵤ) being 5% more than a first detected force peak value (F₁) and a lower limit value (L_{I}) being 5% less than the first detected force peak value (F₁).

12. The method of claim 10 or 11, wherein
an end of the cardiopulmonary resuscitation is determined if, within a predefined interval after a last force peak (P₃) determined as being a force peak of the cardiopulmonary resuscitation, there are no force peaks, or the consecutive force peaks are outside the predefined force peak value range (R_{FP}) and/or outside the predefined force peak interval range.

13. The method of anyone of claims 10 to 12, wherein
occurrence of the cardiopulmonary resuscitation is stored by the controller (5).

14. The method of claim 13, wherein
a duration of the cardiopulmonary resuscitation is detected and stored by the controller (5).

15. The method of anyone of claims 10 to 13, wherein
occurrence of the cardiopulmonary resuscitation and/or a duration of the cardiopulmonary resuscitation are/is transferred to a hospital's Electronic Medical Record system.

## Patentansprüche

1. Operationstisch (1), der Folgendes beinhaltet:
eine Basis (2),
eine Tischplatte (3), die an der Basis (2) angebracht ist,
einen Kraftaufnehmer (4) und
eine Steuerung (5), wobei
der Kraftaufnehmer (4) zum Erkennen einer auf die Tischplatte (3) ausgeübten Kraft und zum Übertragen der Kraft an die Steuerung (5) konfiguriert ist und
die Steuerung (5) zum Auswerten der auf die Tischplatte (3) ausgeübten Kraft und zum Ausführen einer vordefinierten Routine zum Bestimmen kardiopulmonaler Wiederbelebung für einen auf der Tischplatte (3) liegenden Patienten durch Unterscheiden der durch die kardiopulmonale Wiederbelebung verursachten Kräfte von anderen auf die Tischplatte (3) ausgeübten Kräften konfiguriert ist.

2. Operationstisch (1) nach Anspruch 1, wobei
die vordefinierte Routine zum Abbrechen der Auswertung während Bewegung des Operationstisches (1) konfiguriert ist.

3. Operationstisch (1) nach Anspruch 2, wobei
die vordefinierte Routine konfiguriert ist zum Bestimmen ausgeübter Kräfte als Kräfte der kardiopulmonalen Wiederbelebung, wenn Kraftspitzenwerte innerhalb eines vordefinierten Kraftspitzenwertebereichs (R_{FP}) liegen und ein Kraftspitzenintervall innerhalb eines vordefinierten Kraftspitzenintervallbereichs liegt.

4. Operationstisch (1) nach Anspruch 3, wobei
der vorbestimmte Kraftspitzenwertebereich (R_{FP}) von einem oberen Grenzwert (Lᵤ), der 5 % mehr als ein erster erkannter Kraftspitzenwert ist, und einem unteren Grenzwert (Lᵢ), der 5 % weniger als der erste erkannte Kraftspitzenwert (F₁) ist, definiert wird.

5. Operationstisch (1) nach Anspruch 3 oder 4, wobei
der vorbestimmte Kraftspitzenintervallbereich von einem Bereich von 80 bis 120 Kraftspitzen/Minute gebildet wird.

6. Operationstisch (1) nach einem der vorhergehenden Ansprüche, wobei
der Kraftsensor (4) in der Tischplatte (3) angeordnet ist.

7. Operationstisch (1) nach einem der Ansprüche 1 bis 5, wobei
der Kraftsensor (4) in der Basis (2) angeordnet ist.

8. Operationstisch (1) nach einem der vorhergehenden Ansprüche, wobei
der Kraftsensor (4) als ein Sensor konfiguriert ist, der der Steuerung (5) zusätzlich Belastungsdaten der Tischplatte (3) bereitstellt.

9. Operationstisch (1) nach einem der vorhergehenden Ansprüche, wobei
die Steuerung (5) zum Speichern des Stattfindens der kardiopulmonalen Wiederbelebung konfiguriert ist.

10. Verfahren zur Erkennung kardiopulmonaler Wiederbelebung durch einen Operationstisch (1) nach einem der vorhergehenden Ansprüche, das die folgenden Schritte aufweist:
Erkennen mehrerer aufeinanderfolgender Kraftspitzen (P1, P2, P3, P1', P2', P3') durch den Kraftsensor (4); und
Bestimmen der aufeinanderfolgenden Kraftspitzen (P1, P2, P3) als kardiopulmonale Wiederbelebung, wenn die Kraftspitzenwerte innerhalb eines vordefinierten Kraftspitzenwertebereichs (RFP) liegen und die Kraftspitzen innerhalb eines vordefinierten Kraftspitzenintervallbereichs liegen.

11. Verfahren nach Anspruch 10, wobei
der vorbestimmte Kraftspitzenwertebereich (R_{FP}) von einem oberen Grenzwert (Lᵤ), der 5 % mehr als ein erster erkannter Kraftspitzenwert (F₁) ist, und einem unteren Grenzwert (Lᵢ), der 5 % weniger als der erste erkannte Kraftspitzenwert (F₁) ist, definiert wird.

12. Verfahren nach Anspruch 10 oder 11, wobei
ein Ende der kardiopulmonalen Wiederbelebung bestimmt wird, wenn es innerhalb eines vordefinierten Intervalls nach einer letzten Kraftspitze (P₃), die als eine Kraftspitze der kardiopulmonalen Wiederbelebung bestimmt wird, keine Kraftspitzen gibt oder die aufeinanderfolgenden Kraftspitzen außerhalb des vordefinierten Kraftspitzenwertebereichs (R_{FP}) und/oder außerhalb des vordefinierten Kraftspitzenintervallbereichs liegen.

13. Verfahren nach einem der Ansprüche von 10 bis 12, wobei
Stattfinden der kardiopulmonalen Wiederbelebung von der Steuerung (5) gespeichert wird.

14. Verfahren nach Anspruch 13, wobei
eine Dauer der kardiopulmonalen Wiederbelebung von der Steuerung (5) erkannt und gespeichert wird.

15. Verfahren nach einem der Ansprüche 10 bis 13, wobei
das Stattfinden der kardiopulmonalen Wiederbelebung und/oder eine Dauer der kardiopulmonalen Wiederbelebung an das elektronische Gesundheitsaktensystem eines Krankenhauses übertragen wird/werden.

## Revendications

1. Table chirurgicale (1) comprenant :
une base (2),
un dessus de table (3) attaché à la base (2),
un capteur de force (4), et
un contrôleur (5), dans laquelle
le capteur de force (4) est configuré pour détecter une force exercée sur le dessus de table (3) et transmettre la force au contrôleur (5), et
le contrôleur (5) est configuré pour évaluer la force exercée sur le dessus de table (3) et exécuter une routine prédéfinie pour déterminer une réanimation cardiopulmonaire pour un patient reposant sur le dessus de table (3) en distinguant des forces causées par la réanimation cardiopulmonaire parmi d'autres forces exercées sur le dessus de table (3).

2. Table chirurgicale (1) selon la revendication 1, dans laquelle
la routine prédéfinie est configurée pour arrêter l'évaluation pendant un mouvement de la table chirurgicale (1).

3. Table chirurgicale (1) selon la revendication 2, dans laquelle
la routine prédéfinie est configurée pour déterminer des forces exercées comme des forces de la réanimation cardiopulmonaire lorsque des valeurs de pics de force sont dans une plage prédéfinie de valeurs de pics de force (R_{FP}) et qu'un intervalle de pics de force est dans une plage prédéfinie d'intervalle de pics de force.

4. Table chirurgicale (1) selon la revendication 3, dans laquelle
la plage de valeurs de pics de force prédéterminée (R_{FP}) est définie par une valeur limite supérieure (Lᵤ) étant de 5 % supérieure à une première valeur de pic de force détectée et une valeur limite inférieure (L_{I}) étant de 5 % inférieure à la première valeur de pic de force détectée (F₁).

5. Table chirurgicale (1) selon la revendication 3 ou 4, dans laquelle
la plage d'intervalle de pics de force prédéterminée est formée par une plage de 80 à 120 pics de force/minute.

6. Table chirurgicale (1) selon l'une quelconque des revendications précédente, dans laquelle
le détecteur de force (4) est arrangé dans le dessus de table (3).

7. Table chirurgicale (1) selon l'une quelconque des revendications 1 à 5, dans laquelle
le capteur de force (4) est arrangé dans la base (2).

8. Table chirurgicale (1) selon l'une quelconque des revendications précédentes, dans laquelle
le détecteur de force (4) est configuré comme un capteur fournissant en plus des données de charge du dessus de table (3) au contrôleur (5).

9. Table chirurgicale (1) selon l'une quelconque des revendications précédentes, dans laquelle
le contrôleur (5) est configuré pour stocker l'occurrence de la réanimation cardiopulmonaire.

10. Procédé de détection de réanimation cardiopulmonaire par une table chirurgicale (1) selon l'une quelconque des revendications précédentes, comprenant les étapes consistant à :
détecter plusieurs pics de force consécutifs (P₁, P₂, P₃, P₁', P₂', P₃') par le détecteur de force (4) ; et
déterminer les pics de force consécutifs (P₁, P₂, P₃) comme une réanimation cardiopulmonaire si les valeurs de pics de force sont dans une plage prédéfinie de valeurs de pics de force (R_{FP}) et que les pics de force sont dans une plage prédéfinie d'intervalle de pics de force.

11. Procédé selon la revendication 10, dans lequel
la plage prédéfinie de valeurs de pics de force (R_{FP}) est définie par une valeur limite supérieure (Lᵤ) étant de 5 % supérieure à une première valeur de pic de force détectée (F₁) et une valeur limite inférieure (L_{I}) étant de 5 % inférieure à la première valeur de pic de force détectée (F₁).

12. Procédé selon la revendication 10 ou 11, dans lequel
une fin de la réanimation cardiopulmonaire est déterminée si, dans un intervalle prédéfini après un dernier pic de force (P₃) déterminé comme étant un pic de force de la réanimation cardiopulmonaire, il n'y a pas de pic de force, ou bien les pics de force consécutifs sont hors de la plage prédéfinie de valeurs de pics de force (R_{FP}) et/ou hors de la plage prédéfinie d'intervalle de pics de force.

13. Procédé selon l'une quelconque des revendications 10 à 12, dans lequel
une occurrence de la réanimation cardiopulmonaire est stockée par le contrôleur (5).

14. Procédé selon la revendication 13, dans lequel
une durée de la réanimation pulmonaire est détectée et stockée par le contrôleur (5).

15. Procédé selon l'une quelconque des revendications 10 à 13, dans lequel
une occurrence de la réanimation cardiopulmonaire et/ou une durée de la réanimation cardiopulmonaire sont/est transférée(s) à un système d'enregistrement médical électronique de l'hôpital.
